# EUROPÄISCHE PATENTSCHRIFT

(11) **EP 3 656 332 B1**
(45) Veröffentlichungstag und Bekanntmachung des Hinweises auf die Patenterteilung: **07.06.2023**
(21) Anmeldenummer: 19170840.3
(22) Anmeldetag: 24.04.2019
(51) Int. Cl.: A61B 18/22, A61B 18/00, G02B 6/36

(54) **HANDSTÜCK ZUR HANDHABUNG EINER LICHTLEITERFASER BEI EINEM LASERCHIRURGISCHEN EINGRIFF**
HANDPIECE FOR HANDLING AN OPTICAL FIBRE IN LASER SURGERY
PIÈCE À MAIN PERMETTANT DE MANIPULER UNE FIBRE OPTIQUE DANS UNE INTERVENTION CHIRURGICALE AU LASER

(30) Priorität: 22.11.2018 DE 202018106650 U
(43) Veröffentlichungstag der Anmeldung: 27.05.2020
(73) Patentinhaber: LISA Laser Products GmbH, 37191 Katlenburg-Lindau (DE)
(72) Erfinder: Hartkopf-Ceylan, Kay-Siebo, 37154 Northeim (DE)
(74) Vertreter: Holz, Christian

(56) Entgegenhaltungen:
- US-A1- 2003 199 860
- US-A1- 2009 093 800
- US-A1- 2014 005 642
- US-A1- 2016 262 932

## Beschreibung

### TECHNISCHES GEBIET DER ERFINDUNG

Die Erfindung betrifft ein Handstück zur Handhabung einer Lichtleiterfaser bei einem laserchirurgischen Eingriff mit einem längs einer Hauptachse gestreckt ausgebildeten Griffkörper, einem an einem Ende des Griffkörpers angesetzten Führungsrohr mit einer geraden Grundform, wobei ein Lumen des Führungsrohrs mit einem längs der Hauptachse durch den Griffkörper verlaufenden Durchgangsloch fluchtet und mit einer im Bereich des Durchgangslochs in den Griffkörpern angeordneten Halteeinrichtung für die sich durch das Durchgangsloch und das Lumen des Führungsrohrs erstreckende Lichtleiterfaser. Ein solches Handstück kann beispielsweise zur Handhabung einer Lichtleiterfaser verwendet werden, mit der Laserlicht von einem Laser an den Ort eines chirurgischen Eingriffs geleitet wird, um wasserhaltiges Gewebe oder andere Objekte zu erhitzen. Dieses Erhitzen kann der Verödung von Gewebe, dem Schneiden von Gewebe, dem Verdampfen von Gewebe, dem Zertrümmern von Objekten und dergleichen dienen. Das Laserlicht, das mit der Lichtleiterfaser geleitet wird, kann beispielsweise eine Wellenlänge im Bereich von 2 µm aufweisen, bei der Wasser eine besonders hohe Absorption aufweist.

### STAND DER TECHNIK

Handstücke zur Handhabung einer Lichtleiterfaser unterliegen verschiedenen Anforderungen. Hierzu zählt ihre einfache Sterilisierbarkeit, insbesondere bei wiederholter Verwendung, eine einfache und reversible Fixierung der Lichtleiterfaser ihrer Länge nach in dem Handstück, um die Lichtleiterfaser bei Verbrauch ihrer bisherigen Spitze vorschieben und abschneiden zu können, ihre Anpassbarkeit an unterschiedliche räumliche Lagen des Ort des chirurgischen Eingriffs, ihre Einsetzbarkeit während gleichzeitiger MRT-Abbildung des Orts des chirurgischen Eingriffs und dergleichen.

Für Katheter sind Führungsdrähte aus Nitinol bekannt, die für die Platzierung des Katheters in dessen Lumen angeordnet werden. Dabei werden die besonderen Elastizitätseigenschaften von Nitinol unterhalb und auch im Bereich seiner Übergangstemperatur ausgenutzt.

Bei Stents aus Nitinol wird die Eigenschaft von Nitinol als Formgedächtnislegierung ausgenutzt. Die Stents werden in einem radial komprimierten Zustand implantiert und dehnen sich dann bis auf ihren Ausgangsdurchmesser zurück aus, wenn sie beim Erreichen der Körpertemperatur die Übergangstemperatur der hierfür speziell abgestimmten Nitinol-Legierung überschreiten.

Es sind aktive Endoskope bekannt, deren Form mit Hilfe von Elementen aus Formgedächtnislegierungen willkürlich veränderbar ist.

Ein Handstück zur Handhabung einer Lichtleiterfaser bei einem laserchirurgischen Eingriff ist aus der US 2014/0005642 A1 bekannt. Das Handstück weist einen längs einer Hauptachse gestreckt ausgebildeten Griffkörper auf, der einen längs der Hauptachse verschiebbaren Betätigungsmechanismus aufnimmt und der an seinem proximalen Ende mit einer Kappe verschlossen ist. An den Betätigungsmechanismus ist ein flexibles Führungsrohr für die Lichtleiterfaser angesetzt, das längs der Hauptachse über ein proximales Ende des Griffkörpers vorsteht. Mit Hilfe des Betätigungsmechanismus kann das flexible Führungsrohr gegenüber dem Griffkörper vorgeschoben und zurückgezogen werden. Das flexible Führungsrohr kann ein Formgedächtnismaterial aufweisen, beispielsweise Nitinol. Die Lichtleiterfaser kann z. B. durch Verkleben oder eine Halteeinrichtung im Bereich der Kappe des Griffkörpers fixiert sein, so dass durch das Verschieben des flexiblen Führungsrohrs mit der Betätigungsvorrichtung das flexible Führungsrohr auch gegenüber der Lichtleiterfaser verschoben wird. Durch die Relativverschiebung des flexiblen Führungsrohrs gegenüber dem Griffkörper soll die Lichtleiterfaser schrittweise krümmbar oder alternativ aus einer gekrümmten Form streckbar sein. Dabei sollen beim Verschieben des flexiblen Führungsrohrs gegenüber dem Griffkörper Kräfte auf das flexible Führungsrohr ausgeübt werden.

Aus der US 2009/0093800 A1 ist ein Handstück zur Handhabung einer Lichtleiterfaser mit einem Griffkörper und einem an dem Griffkörper angesetzten flexiblen Führungsrohr bekannt. Das flexible Führungsrohr ist über den größten Teil seiner Länge in einer an den Griffkörper angesetzte Kanüle geführt. Der Griffkörper kann aus rostfreiem Stahl, Titan oder einem thermoplastischen Kunststoff ausgebildet sein. Die Kanüle kann aus Titan oder rostfreiem Stahl ausgebildet sein. Das Führungsrohr ist aus einer Formgedächtnislegierung wie Nitinol ausgebildet. Der über die Kanüle überstehende Teil des Führungsrohrs ist viertelkreisförmig gekrümmt.

Aus der US 2016/0262932 A1 ist ein Handstück zur Handhabung einer Lichtleiter bei einem chirurgischen Eingriff bekannt, bei dem an einem Ende eines Griffkörpers ein flexibles Führungsrohr aus Kunststoff für die Lichtleiterfaser angesetzt ist. Das flexible Führungsrohr ist mit einem gewünschten Krümmungsradius vorgeformt. Das Führungsrohr kann aus einem Formgedächtnismaterial, insbesondere einem Formgedächtnispolymer, bestehen. Es kann durch ein innen oder außen angeordnetes Material, insbesondere rostfreiem Stahl, verstärkt sein. In das flexible Führungsrohrs aus Kunststoff kann neben der Lichtleiterfaser ein weiteres Führungselement aus einem weiteren Material, wie beispielsweise rostfreiem Stahl oder Nitinol eingesetzt sein, das die Lichtleiterfaser umgibt und längs der Hauptachse der Anordnung verschoben werden kann, um das flexible Führungsrohr aus Kunststoff und die Lichtleiterfaser zu strecken, wenn es mit Hilfe eines an dem Griffkörper angeordneten Schiebeelements vorwärts bewegt wird.

Aus der US 2003/0199860 A1 ist eine Kathetervorrichtung mit einer Lichtleiterfaser bekannt, die die Merkmale des Oberbegriffs des Patentanspruchs 1 aufweist. Das distale Ende der Lichtleiterfaser ist in einem Rohr mit einem scharfkantigen oder injektionsnadelförmigen Ende angeordnet. Das distale Ende der Lichtleiterfaser und das Führungsrohr sind so ausgebildet, dass durch Reflektion an einer Metalloberfläche Laserenergie unter einem Winkel von 80° bis 90° relativ zur Längsachse der optischen Faser und des Führungsrohrs abgestrahlt wird. Das Führungsrohr ist an den Griffkörper eines Handstücks angesetzt. Weiterhin ist an den Griffkörper ein Kühlflüssigkeitszufuhrstutzen angesetzt, der mit dem Lumen des Führungsrohrs kommuniziert und die dadurch verlaufende Lichtleiterfaser kühlt. Dabei ist die Lichtleiterfaser in dem Griffstück durch Klebstoff fixiert und so auf der dem Führungsrohr abgekehrten Seite des Kühlflüssigkeitszufuhrstutzens gegenüber dem Griffkörper abgedichtet. Bei einer anderen aus der US 2003/0199860 A1 bekannten Kathetervorrichtung mit einer Lichtleiterfaser ist ein Führungsrohr aus einer Nickel-Titan-Formgedächtnislegierung ausgebildet und in einem distalen Endbereich warm in eine gekrümmte Grundform verformt.

### AUFGABE DER ERFINDUNG

Der Erfindung liegt die Aufgabe zugrunde, ein Handstück zur Handhabung einer Lichtleiterfaser bei einem laserchirurgischen Eingriff aufzuzeigen, das trotz einer hohen Funktionalität einfach und sicher sterilisierbar ist, um seine wiederholte Verwendung zu ermöglichen.

### LÖSUNG

Die Aufgabe der Erfindung wird durch ein Handstück mit den Merkmalen des unabhängigen Anspruchs 1 gelöst. Die abhängigen Ansprüche betreffen bevorzugte Ausführungsformen des erfindungsgemäßen Handstücks.

### BESCHREIBUNG DER ERFINDUNG

Bei einem erfindungsgemäßen Handstück zur Handhabung einer Lichtleiterfaser während eines laserchirurgischen Eingriffs mit einem längs einer Hauptachse gestreckt ausgebildeten Griffkörper, einem an einem Ende des Griffkörpers angesetzten Führungsrohr, wobei ein Lumen des Führungsrohrs mit einem längs der Hauptachse durch den Griffkörper verlaufenden Durchgangsloch fluchtet, und einer im Bereich des Durchgangslochs in dem Griffkörper angeordneten Halteeinrichtung für die sich durch das Durchgangsloch und das Lumen des Führungsrohrs hindurch erstreckende Lichtleiterfaser ist das Führungsrohr aus einer Formgedächtnislegierung ausgebildet, die eine Übergangstemperatur zwischen 50 °C und 120 °C aufweist, wobei das Führungsrohr eine gerade Grundform aufweist, die es nach einer unterhalb der Übergangstemperatur erfolgten plastischen Verformung bei Überschreiten der Übergangstemperatur wieder annimmt.

Unter dem Lumen des Führungsrohrs wird hier das freie Durchgangsloch oder das freie Innenvolumen des Führungsrohrs verstanden, das von dem Führungsrohr in radialer Richtung von der Hauptachse weg begrenzt ist.

Bei dem erfindungsgemäßen Handstück befindet sich das Führungsrohr bei Raumtemperatur und auch bei der Körpertemperatur eines Menschen signifikant unterhalb der Übergangstemperatur der Formgedächtnislegierung, aus dem das Führungsrohr ausgebildet ist. Das Führungsrohr ist daher in dem Temperaturbereich seiner Verwendung bei einem laserchirurgischen Eingriff grundsätzlich formstabil und es weist eine relativ hohe Steifigkeit, es ist also nicht etwa superelastisch. Das Führungsrohr kann in dem Temperaturbereich seiner Verwendung aber kalt plastisch verformt werden, um ihm einen gewünschten räumlichen Verlauf zu geben. Bei einem typischen geringen Außendurchmesser des Führungsrohrs von wenigen Millimetern und einer typischen Wandstärke des Führungsrohrs von wenigen Zehntelmillimetern sind für die plastische Verformung des Führungsrohrs keine großen Kräfte erforderlich. Das Führungsrohr lässt sich damit leicht in eine Form biegen, in der es geeignet ist, die Lichtleiterfaser mit einer gewünschten Ausrichtung an den Ort des laserchirurgischen Eingriffs heranzuführen. Bei seiner planmäßigen Verwendung geht die dem Führungsrohr durch plastische Verformung gegebene Form nicht verloren. Sie wird aber beseitigt, wobei das Führungsrohr seine gerade Grundform wieder annimmt, wenn das Führungsrohr über seine Überganstemperatur, die in den Bereich von 50 °C bis 120 °C fällt, hinaus erwärmt wird. Die konkrete Übergangstemperatur der Formgedächtnislegierung, aus dem das Führungsrohr ausgebildet wird, ist bei dem erfindungsgemäßen Handstück einerseits so festzulegen, dass sie bei der geplanten Verwendung des Handstücks nicht erreicht wird. Andererseits wird sie vorzugsweise so gewählt, dass sie bei einer planmäßigen Reinigung und Sterilisation des erfindungsgemäßen Handstücks überschritten wird. Die gerade Grundform des Führungsrohrs erleichtert seine Reinigung. Umgekehrt zeigt die wieder erreichte gerade Grundform des Führungsrohrs an, dass es bis auf eine Temperatur oberhalb seiner Übergangstemperatur erwärmt wurde. Dies ist ein Indiz für seine planmäßig durchgeführte Reinigung bei erhöhter Temperatur.

Das Führungsrohr des erfindungsgemäßen Handstücks erstreckt sich vorzugsweise mit seiner geraden Grundform längs der Hauptachse, so dass es das mit ihm fluchtende Durchgangsloch durch den Griffkörper geradlinig verlängert.

Vorzugsweise liegt die Übergangstemperatur der Formgedächtnislegierung, aus dem das Führungsrohr des erfindungsgemäßen Handgriffs ausgebildet ist, in einem Bereich zwischen 60 und 90 °C. Typischerweise besteht beim Erreichen einer Temperatur von 60 °C die Gefahr, dass biologisches Gewebe bereits durch einen relativ kurzzeitigen Kontakt mit einem auf dieser Temperatur befindlichen Gegenstand ernsthaft geschädigt wird. Entsprechend ist es üblich, dass ein Laser, an dem die Lichtfaser bei dem laserchirurgischen Eingriff angekoppelt ist, und das Laserlicht in die Lichtleiterfaser einkoppelt, beim Erreichen dieser Temperatur am Ende der Lichtleiterfaser abgeschaltet wird. Damit tritt bei der geplanten Verwendung des erfindungsgemäßen Handstücks keine Temperatur des Führungsrohrs von 60 °C oder mehr auf. Eine Temperatur des Führungsrohrs von bis zu 90 °C wird hingegen schnell in einem Wasserbad erreicht, das sich auf einer Temperatur nahe des Siedepunkts von Wasser befindet. Konkret die Übergangstemperatur der Formgedächtnislegierung in einem Bereich von 62 bis 65 °C liegen, womit die anfängliche Übergangstemperatur der Formgedächtnislegierung gemeint ist, die sich bekanntermaßen bei wiederholter ausgeprägter Kaltverformung des Führungsrohrs erhöhen kann.

An den Griffkörper des erfindungsgemäßen Handstücks kann ein Kühlflüssigkeitszufuhrstutzen angesetzt sein, der über das Durchgangsloch mit dem Lumen des Führungsrohrs kommuniziert. An diesen Kühlflüssigkeitszufuhrstutzen kann ein Kühlflüssigkeitsschlauch angeschlossen werden, um Kühlflüssigkeit, insbesondere Wasser, zuzuführen, die dann längs der Lichtleiterfasern durch das Führungsrohr an den Ort des jeweiligen chirurgischen Eingriffs strömt. Die Kühlflüssigkeit kühlt dabei das Führungsrohr und die Lichtleiterfaser sowie auch an den Ort des chirurgischen Eingriffs angrenzendes Gewebe.

Der Griffkörper des erfindungsgemäßen Handstücks kann zwei um die Hauptachse herum miteinander verschraubte Griffteile aufweisen. Zwischen den beiden Griffteilen kann dabei eine ringförmige elastische Dichtung angeordnet sein, die beim Zusammenschrauben der beiden Griffteile zu der Hauptachse hin zusammengedrückt wird und sich als Teil der Halteeinrichtung an die durch sie hindurchgeführte Lichtleiterfaser anlegt. Die an der Lichtleiterfaser anliegende Dichtung hält diese mit einer elastischen Klemmkraft fest, die mit dem Zusammenschrauben der beiden Griffteile anwächst. Zugleich dichtet die Dichtung das Durchgangsloch um die Lichtleiterfaser herum ab. Weiterhin führt die elastische Dichtung auch dazu, dass die beiden Griffteile nicht unbeabsichtigt auseinandergeschraubt werden, auch wenn sie noch nicht hart gegeneinander geschraubt und dabei direkt gegeneinander verspannt sind.

Die mit der ringförmigen Dichtung erreichte Abdichtung des Durchgangslochs um die Lichtleiterfaser ist dann, wenn zugleich ein Kühlflüssigkeitszufuhrstutzen an den Griffkörper des erfindungsgemäßen Handstücks angesetzt ist, auf einer dem Führungsrohr abgekehrten Seite des Kühlflüssigkeitszufuhrstutzens angeordnet. Sie verhindert so das Austreten der Kühlflüssigkeit durch das Durchgangsloch an dem dem Führungsrohr abgekehrten Ende des Griffkörpers. Zudem verhindert sie bei geeigneter Ausbildung und Anordnung das Austreten der Kühlflüssigkeit zwischen den beiden Griffteilen des Griffkörpers.

Das Führungsrohr des erfindungsgemäßen Handstücks kann in einen über den Griffkörper überstehenden Führungsrohraufnahmestutzen eingesteckt sein. Dabei kann das Führungsrohr in dem Führungsrohraufnahmestutzen lösbar oder dauerhaft verankert sein. Beides kann zum Beispiel durch Reibschluss realisiert sein. Der Führungsrohraufnahmestutzen stabilisiert das Führungsrohr an dem Übergang in den Griffkörper und verhindert ein Abknicken des Führungsrohrs an diesem Übergang.

Auf dem Führungsrohr des erfindungsgemäßen Handstücks kann ein elastischer Absaugschlauch angeordnet sein, dessen Innendurchmesser größer ist als der Außendurchmesser des Führungsrohrs, so dass zwischen dem Führungsrohr und dem Absaugschlauch ein Ringraum verbleibt. Über diesen Ringraum kann zum Beispiel Rauch aus einem Operationsbereich vor dem vorderen Ende des Führungsrohrs abgesaugt werden.

Um einen Unterdruck an den Absaugschlauch anzulegen, der für eine solche Absaugung erforderlich ist, kann ein mit dem Absaugschlauch kommunizierender Absaugstutzen vorgesehen sein, an den eine Absaugleitung anschließbar ist. Dieser Absaugstutzen kann an den Griffkörper oder an einen über das Führungsrohr aufgeschobenen separaten Absaugverteiler angesetzt sein. Ein solcher Absaugverteiler kann fest mit dem Absaugschlauch auf dem Führungsrohr verbunden sein. Wenn der Absaugstutzen an den Griffkörper angesetzt ist, kann der Absaugschlauch auf einen Absaugschlauchaufsteckstutzen, der im Übergangsbereich in den Griffkörper um das Führungsrohr herum angeordnet ist, aufgeschoben sein.

Die Formgedächtnislegierung, aus der das Führungsrohr des erfindungsgemäßen Handstücks ausgebildet ist, ist insbesondere eine Nickeltitanlegierung, die auch als Nitinol bezeichnet wird. Konkret kann es sich um Nitinol SM 495 handeln, eine zu 54,5 % aus Nickel bestehende Nickeltitanlegierung, deren Übergangstemperatur je nach Vorbehandlung in einen Bereich von 50 bis 80 °C fällt. Konkret kann diese Übergangstemperatur durch geeignete Vorbehandlung etwa 63 bis 64 °C betragen.

Alle elastischen Bestandteile des erfindungsgemäßen Handstücks können aus Silikonkautschuk ausgebildet sein. Silikonkautschuk zeichnet sich durch eine ausreichende Temperaturbeständigkeit aus, um eine thermische Sterilisation unbeschädigt zu überstehen. Zu den Bestandteilen des erfindungsgemäßen Handstücks, die aus Silikonkautschuk ausgebildet sein können, zählen insbesondere die ringförmige elastische Dichtung zwischen den beiden Griffteilen, der Absaugschlauch und auch ein daran angeschlossener Verteiler.

Der Griffkörper bzw. die Griffteile des erfindungsgemäßen Handstücks können aus Polyetheretherketon (PEEK) ausgebildet sein. PEEK ist ein bis über 300 °C temperaturbeständiger Kunststoff. Die Ausbildung des Griffkörpers bzw. der Griffteile aus PEEK ermöglicht auch die Verwendung des erfindungsgemäßen Handstücks innerhalb eines Magnetresonanztomographen. Der Magnetismus von Nitinol ist nur gering, und bei nur geringer Wandstärke des Führungsrohrs besteht auch nicht die Gefahr einer ausgeprägten Erwärmung des Führungsrohrs durch Wirbelströme angeregt durch das in einem MRT herrschende magnetische Wechselfeld.

Für eine Anwendung außerhalb von MRT ist eine Ausbildung des Griffkörpers bzw. der Griffteile aus beschichtetem Aluminium bevorzugt, in dem die magnetischen Wechselfelder in einem MRT Wirbelströme hervorrufen könnten, die zu einer signifikanten Temperaturerhöhung führen würden. Die Beschichtung des Aluminiums erfüllt den Zweck einer Passivierung der Oberfläche des Aluminiums. Die Beschichtung des Aluminiums kann durch Eloxieren erfolgt sein. Bevorzugt ist eine Beschichtung des Aluminiums mit Diamond-like Carbon (DLC), mit dem eine dauerhafte Oberfläche großer Härte erreicht wird. Zudem ist DLC laserbeschriftbar. Die gewünschte Oberflächenhärte des Aluminiums kann zusätzlich dadurch erreicht werden, dass die Oberfläche des Aluminiums vor dem Beschichten glasperlgestrahlt wird. Hierdurch ergibt sich eine Oberflächenhärtung durch Kaltverfestigung, die bei dem Aluminium aber durch die beim Sterilisieren des erfindungsgemäßen Handstücks erreichten Temperaturen nicht wieder verlorengeht. Grundsätzlich kann das Aluminium des Griffkörpers oder der Griffteile auch legierungsgehärtet sein. Eine ausgeprägte Legierungshärtung zum Beispiel mit Kupfer erhöht jedoch den Aufwand, der beim Bearbeiten des Aluminiums zur Ausformung des Griffkörpers bzw. der Griffteile erforderlich ist.

Wenn das erfindungsgemäße Handstück einen Kühlflüssigkeitszufuhrstutzen aufweist, ist er vorzugsweise aus PEEK ausgebildet. Grundsätzlich ist aber auch beispielsweise eine Ausbildung aus Edelstahl möglich. Der Führungsrohraufnahmestutzen des erfindungsgemäßen Handstücks ist falls vorhanden vorzugsweise aus PEEK oder Edelstahl ausgebildet, und der Absaugstutzen des erfindungsgemäßen Handstücks ist falls vorhanden vorzugsweise aus PEEK oder Silikonkautschuk ausgebildet. Die genannten Materialien sind sterilisierbar und zumindest im Umfang der genannten Bauteile auch bei Verwendung in einem MRT unproblematisch.

Vorteilhafte Weiterbildungen der Erfindung ergeben sich aus den Ansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Ansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Ansprüche ist ebenfalls abweichend von den gewählten Rückbeziehungen der Ansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Ansprüche kombiniert werden. Ebenso können in den Ansprüchen aufgeführte Merkmale für weitere Ausführungsformen der Erfindung entfallen.

Die in den Ansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Anschluss die Rede ist, ist dies so zu verstehen, dass genau ein Anschluss, zwei Anschlüsse oder mehr Anschlüsse vorhanden sind. Die in den Ansprüchen angeführten Merkmale können durch andere Merkmale ergänzt werden oder die einzigen Merkmale sein, die das jeweilige Handstück aufweist.

Die in den Ansprüchen enthaltenen Bezugszeichen stellen keine Beschränkung des Umfangs der durch die Ansprüche geschützten Gegenstände dar. Sie dienen lediglich dem Zweck, die Ansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: ist eine radiale Draufsicht auf eine Ausführungsform des erfindungsgemäßen Handstücks.
- **Fig. 2**: ist ein Längsschnitt durch das Handstück gemäß Fig. 1 entlang der in Fig. 1 eingezeichneten Schnittlinie A-A.
- **Fig. 3**: ist eine perspektivische Ansicht des erfindungsgemäßen Handstücks nach plastischer Verformung seines Führungsrohrs in eine frei gebogene Form und
- **Fig. 4**: ist eine perspektivische Ansicht des erfindungsgemäßen Handstücks gemäß Fig. 3 nach heißer Reinigung des Handstücks bei einer Temperatur oberhalb einer Übergangstemperatur einer Formgedächtnislegierung, aus dem das Führungsrohr ausgebildet ist.

### FIGURENBESCHREIBUNG

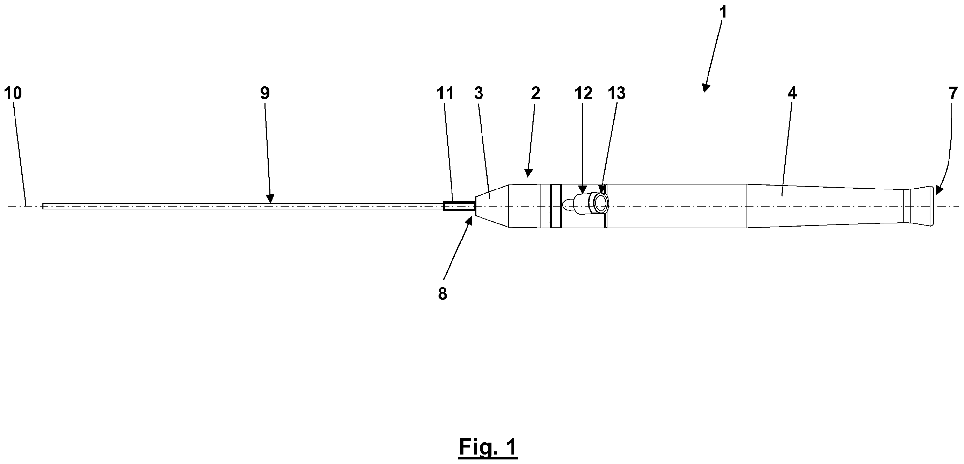

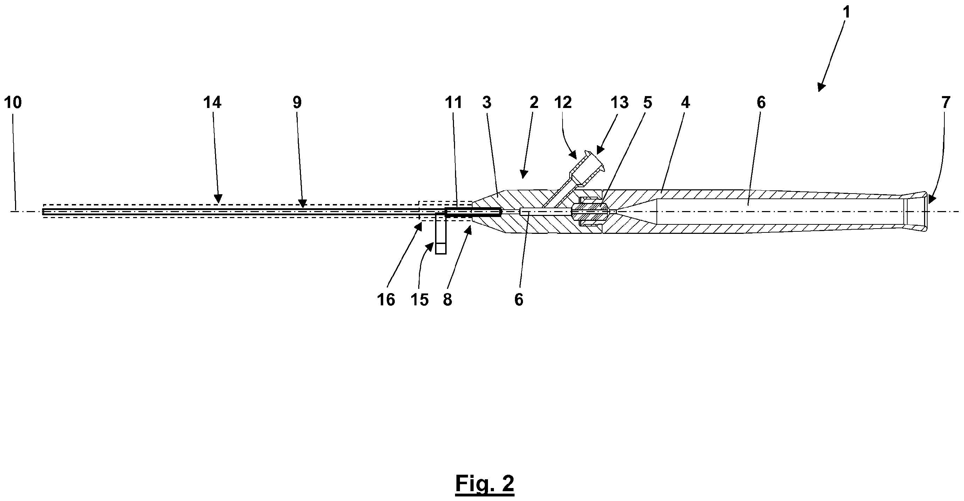

Das in den **Fig. 1** **und** **2** dargestellte Handstück 1 weist einen Griffkörper 2 auf, der aus zwei Griffteilen 3 und 4 zusammengeschraubt ist. Zwischen den Griffteilen 3 und 4 ist eine ringförmige elastische Dichtung 5 angeordnet. Die Dichtung 5 wird beim Zusammenschrauben der Griffteile 3 und 4 zusammengedrückt und sie legt sich dadurch mit einer Haltekraft an einer hier nicht dargestellten, durch sie hindurchgeführten Lichtleiterfaser an, zu deren Handhabung das Handstück 1 vorgesehen ist. Die Lichtleiterfaser verläuft durch ein Durchgangsloch 6 in dem Griffkörper 2. Dieses Durchgangsloch 6 wird durch die Dichtung 5 zwischen den Griffteilen 3 und 4 unterteilt und um die Lichtleiterfaser herum abgedichtet. Die Dichtung 5 dichtet dabei auch die Griffteile 3 und 4 gegeneinander ab. Zu einem hinteren Ende 7 des Griffkörpers 2 hin erweitert sich das Durchgangsloch 6, so dass die Lichtleiterfaser hier mit Spiel durch den Griffkörper 2 verläuft und an dem hinteren Ende 7 gegen Abknicken geschützt ist. An einem dem hinteren Ende 7 gegenüberliegenden vorderen Ende 8 des Griffkörpers 2 ist ein Führungsrohr 9 für die Lichtleiterfaser angesetzt, dessen Lumen mit dem Durchgangsloch 6 fluchtet. Das Führungsrohr 9 weist eine gerade Grundform auf, in der es geradlinig auf einer Hauptachse 10 verläuft, längs der der Griffkörper 2 gestreckt ausgebildet ist. An dem vorderen Ende 8 des Griffkörpers 2, bei seinem Übergang in den Griffkörper 2, ist das Führungsrohr 9 durch einen Führungsrohraufnahmestutzen 11 verstärkt. An dem vorderen Griffteil 3 ist ein Kühlflüssigkeitszufuhrstutzen 12 angesetzt, der mit dem Durchgangsloch 6 auf der Vorderseite der Dichtung 5 kommuniziert und über den eine Kühlflüssigkeit indiziert werden kann, die dann längs der Lichtleiterfaser durch das Führungsrohr 9 strömt. Der Kühlflüssigkeitszufuhrstutzen 12 ist mit einem standardisierten Spritzenanschluss 13 versehen.

Nur in Fig. 2 und nur schematisch ist mit gestrichelten Linien ein auf dem Führungsrohr 9 angeordneter Absaugschlauch 14 dargestellt, dessen Innendurchmesser größer als der Außendurchmesser des Führungsrohrs 9 ist, so dass durch Absaugen an einem an den Absaugschlauch 14 angesetzten Absaugstutzen 15 zum Beispiel Rauchgas aus einem vor dem Führungsrohr 19 liegenden Bereich abgesaugt werden kann. Der Absaugstutzen ist an einen Verteiler 16 angesetzt, an den auch der Absaugschlauch 14 angesetzt ist. Der Absaugschlauch 14 ist elastisch und besteht insbesondere aus Silikonkautschuk. Er kann daher Formänderungen des Führungsrohrs 9 mitmachen. Solche Formänderungen des Führungsrohrs 9 sind durch dessen Ausbildung aus einer Formgedächtnislegierung mit einer Übergangstemperatur von etwa 65 °C nicht nur im elastischen sondern auch im plastischen Bereich möglich.

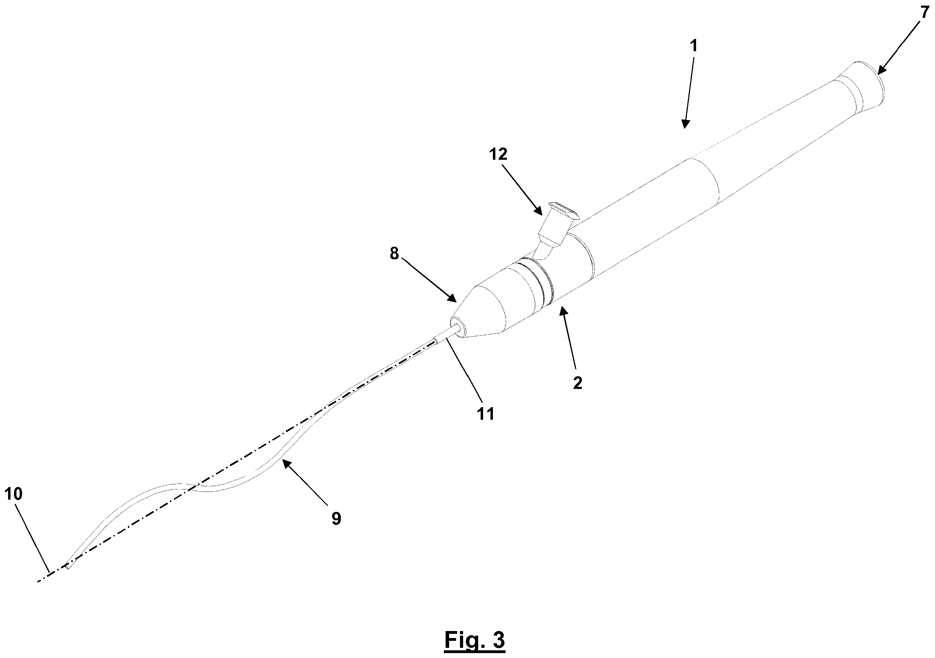

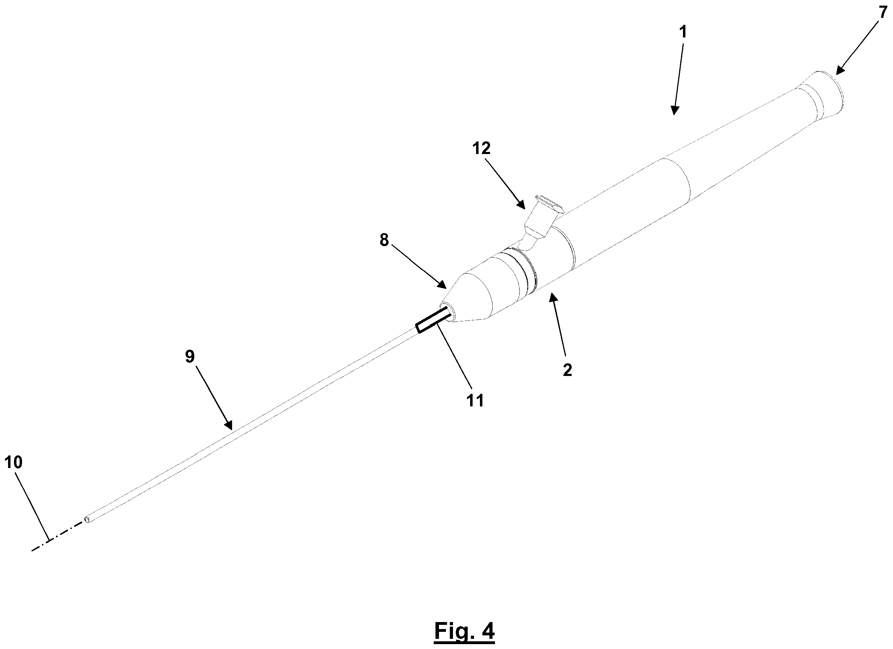

**Fig. 3** zeigt das erfindungsgemäße Handstück 1 mit in eine frei gewählte Form plastisch gebogenem Führungsrohr 9. Mit dieser Form des Führungsrohrs 9 kann die Verwendung des Handstücks 1 zur Handhabung der Lichtleiterfaser bei einem laserchirurgischen Eingriff erfolgen. Bei einer sich an den laserchirurgischen Eingriff anschließenden heißen Reinigung und Sterilisation des Handstücks 1 wird die Übergangstemperatur der Formgedächtnislegierung, aus der das Führungsrohr 9 ausgebildet ist, überschritten, so dass sich das Führungsrohr 9 in seine gerade Grundform zurückformt, wie sie **Fig. 4** zeigt. Diese gerade Form erleichtert eine mechanische Reinigung des Führungsrohrs 9 und zeigt zugleich an, dass es nach der Benutzung des Handstücks 1 bereits einer erhöhten Temperatur im Rahmen seiner Reinigung und/oder Sterilisation ausgesetzt wurde.

### BEZUGSZEICHENLISTE

- 1: Handstück
- 2: Griffkörper
- 3: (vorderer) Griffteil
- 4: (hinterer) Griffteil
- 5: Dichtung
- 6: Durchgangsloch
- 7: hinteres Ende
- 8: vorderes Ende
- 9: Führungsrohr
- 10: Hauptachse
- 11: Führungsrohraufnahmestutzen
- 12: Kühlflüssigkeitszufuhrstutzen
- 13: Spritzenanschluss
- 14: Absaugschlauch
- 15: Absaugstutzen
- 16: Verteiler

## Patentansprüche

1. Handstück (1) zur Handhabung einer Lichtleiterfaser bei einem laserchirurgischen Eingriff mit
- einem längs einer Hauptachse (10) gestreckt ausgebildeten Griffkörper (2),
- einem an einem Ende des Griffkörpers (2) angesetzten Führungsrohr (9) mit einer geraden Grundform,
- wobei ein Lumen des Führungsrohrs (9) mit einem längs der Hauptachse (10) durch den Griffkörper (2) verlaufenden Durchgangsloch (6) fluchtet, und
- einer im Bereich des Durchgangslochs (6) in dem Griffkörper (2) angeordneten Halteeinrichtung für die sich durch das Durchgangsloch (6) und das Lumen des Führungsrohrs (9) hindurch erstreckende Lichtleiterfaser,
**dadurch gekennzeichnet, dass** das Führungsrohr (9) aus einer Formgedächtnislegierung ausgebildet ist, die eine Übergangstemperatur zwischen 50 °C und 120 °C aufweist, wobei das Führungsrohr (9) seine gerade Grundform nach einer unterhalb der Übergangstemperatur erfolgten plastischen Verformung bei Überschreiten der Übergangstemperatur wieder annimmt.

2. Handstück (1) nach Anspruch 1, wobei sich das Führungsrohr (9) mit seiner geraden Grundform längs der Hauptachse (10) erstreckt.

3. Handstück (1) nach einem der vorhergehenden Ansprüche, wobei die Übergangstemperatur zwischen 60 und 90 °C liegt.

4. Handstück (1) nach einem der vorhergehenden Ansprüche, wobei an den Griffkörper (2) ein Kühlflüssigkeitszufuhrstutzen (12) angesetzt ist, der über das Durchgangsloch (6) mit dem Lumen des Führungsrohrs (9) kommuniziert.

5. Handstück (1) nach einem der vorhergehenden Ansprüche, wobei der Griffkörper (2) zwei um die Hauptachse (10) herum miteinander verschraubte Griffteile (3, 4) aufweist, wobei optional zwischen den beiden Griffteilen (3, 4) eine ringförmige elastische Dichtung (5) angeordnet ist, die beim Zusammenschrauben der beiden Griffteile (3, 4) zu der Hauptachse (10) hin zusammengedrückt wird und die Teil der Halteeinrichtung für die Lichtleiterfaser ist.

6. Handstück (1) nach Anspruch 5, soweit rückbezogen auf Anspruch 4, wobei die Dichtung (5) auf einer dem Führungsrohr (9) abgekehrten Seite des Kühlflüssigkeitszufuhrstutzen (12) angeordnet ist.

7. Handstück (1) nach einem der vorhergehenden Ansprüche, wobei das Führungsrohr (9) in einen über den Griffkörper (2) überstehenden Führungsrohraufnahmestutzen (11) eingesteckt ist.

8. Handstück (1) nach einem der vorhergehenden Ansprüche, wobei ein elastischer Absaugschlauch (14) auf dem Führungsrohr (9) angeordnet ist, dessen Innendurchmesser größer ist als der Außendurchmesser des Führungsrohrs (9).

9. Handstück (1) nach Anspruch 8, wobei ein mit dem Absaugschlauch (14) kommunizierender Absaugstutzen (15) an den Griffkörper (2) oder an einen über das Führungsrohr (9) aufgeschobenen separaten Absaugverteiler angesetzt ist.

10. Handstück (1) nach einem der vorhergehenden Ansprüche, wobei das Führungsrohr (9) aus Nitinol ausgebildet ist.

11. Handstück (1) nach einem der vorhergehenden Ansprüche, wobei alle elastischen Bestandteile aus Silikonkautschuk ausgebildet sind.

12. Handstück (1) nach einem der vorhergehenden Ansprüche, wobei der Griffkörper (2) oder die Griffteile (3, 4) aus PEEK ausgebildet ist/sind.

13. Handstück (1) nach einem der Ansprüche 1 bis 11, wobei der Griffkörper (2) oder die Griffteile (3, 4) aus beschichtetem Aluminium ausgebildet ist/sind.

14. Handstück (1) nach Anspruch 13, wobei das Aluminium glasperlgestrahlt und/oder mit DLC beschichtet ist.

15. Handstück (1) nach einem der vorhergehenden Ansprüche, wobei
- der Kühlflüssigkeitszufuhrstutzen (12) aus PEEK ausgebildet ist und/oder
- der Führungsrohraufnahmestutzen (11) aus PEEK oder Edelstahl ausgebildet ist und/oder
- der Absaugstutzen (15) aus PEEK oder Silikonkautschuk ausgebildet ist.

## Claims

1. A handpiece (1) for handling an optical fiber in laser surgery, comprising
- a handle body (2) which is elongate along a main axis (10),
- a guide tube (9) which is attached to one end of the handle body (2) and has a straight basic shape,
- a lumen of the guide tube (9) being aligned with a through hole (6) extending through the handle body (2) along the main axis (10), and
- a holding device for the optical fiber extending through the through hole (6) and through the lumen of the guide tube (9), which holding device is arranged in the handle body (2) in the region of the through hole (6),
**characterized in that** the guide tube (9) is formed of a shape memory alloy having a transition temperature between 50°C and 120°C, the guide tube (9), after a plastic deformation that takes place below the transition temperature, returning to its straight basic shape when the transition temperature is exceeded.

2. The handpiece (1) according to claim 1, wherein the straight basic shape of the guide tube (9) extends along the main axis (10).

3. The handpiece (1) according to either of the preceding claims, wherein the transition temperature is between 60 and 90°C.

4. The handpiece (1) according to any of the preceding claims, wherein a cooling liquid supply connector (12) is attached to the handle body (2) and communicates with the lumen of the guide tube (9) via the through hole (6).

5. The handpiece (1) according to any of the preceding claims, wherein the handle body (2) has two handle parts (3, 4) which are screwed together around the main axis (10), wherein an annular elastic seal (5) is optionally arranged between the two handle parts (3, 4), is compressed towards the main axis (10) when the two handle parts (3, 4) are screwed together and is part of the holding device for the optical fiber.

6. The handpiece (1) according to claim 5, when dependent on claim 4, wherein the seal (5) is arranged on a side of the cooling liquid supply connector (12) that faces away from the guide tube (9).

7. The handpiece (1) according to any of the preceding claims, wherein the guide tube (9) is inserted into a guide tube receiving connector (11) projecting beyond the handle body (2).

8. The handpiece (1) according to any of the preceding claims, wherein an elastic suction hose (14) is arranged on the guide tube (9), the inner diameter of which hose is greater than the outer diameter of the guide tube (9).

9. The handpiece (1) according to claim 8, wherein a suction nozzle (15) communicating with the suction hose (14) is attached to the handle body (2) or to a separate suction distributor pushed on over the guide tube (9).

10. The handpiece (1) according to any of the preceding claims, wherein the guide tube (9) is formed of nitinol.

11. The handpiece (1) according to any of the preceding claims, wherein all elastic components are formed of silicone rubber.

12. The handpiece (1) according to any of the preceding claims, wherein the handle body (2) or the handle parts (3, 4) is/are formed of PEEK.

13. The handpiece (1) according to any of claims 1 to 11, wherein the handle body (2) or the handle parts (3, 4) is/are formed of coated aluminum.

14. The handpiece (1) according to claim 13, wherein the aluminum is glass-bead blasted and/or coated with DLC.

15. The handpiece (1) according to any of the preceding claims, wherein
- the cooling liquid supply connector (12) is formed of PEEK and/or
- the guide tube receiving connector (11) is formed of PEEK or stainless steel and/or
- the suction nozzle (15) is formed of PEEK or silicone rubber.

## Revendications

1. Pièce à main (1) permettant de manipuler une fibre optique dans une intervention chirurgicale au laser, comprenant
- un corps de préhension (2) conçu de manière allongée le long d'un axe principal (10),
- un tube de guidage (9) présentant une forme de base droite, fixé en une extrémité du corps de préhension (2),
- une lumière du tube de guidage (9) étant alignée sur un trou de passage (6) s'étendant le long de l'axe principal (10) à travers le corps de préhension (2) et
- un dispositif de fixation, agencé au niveau du trou de passage (6) dans le corps de préhension (2), pour la fibre optique qui s'étend à travers le trou de passage (6) et la lumière du tube de guidage (9),
**caractérisée en ce que** le tube de guidage (9) est conçu en un alliage à mémoire de forme qui présente une température de transition entre 50 °C et 120 °C, le tube de guidage (9) reprenant sa forme de base droite après une déformation plastique ayant lieu sous la température de transition lorsque la température passe au-dessus de la température de transition.

2. Pièce à main (1) selon la revendication 1, le tube de guidage (9) s'étendant avec sa forme de base droite le long de l'axe principal (10).

3. Pièce à main (1) selon l'une quelconque des revendications précédentes, la température de transition étant située entre 60 et 90 °C.

4. Pièce à main (1) selon l'une quelconque des revendications précédentes, une tubulure d'alimentation (12) en liquide de refroidissement étant fixée sur le corps de préhension (2), laquelle tubulure communique avec la lumière du tube de guidage (9) par l'intermédiaire du trou de passage (6).

5. Pièce à main (1) selon l'une quelconque des revendications précédentes, le corps de préhension (2) présentant deux parties de préhension (3, 4) vissées l'une à l'autre autour de l'axe principal (10), un joint élastique annulaire (5) étant éventuellement agencé entre les deux parties de préhension (3, 4), lequel joint est comprimé lors du vissage des deux parties de préhension (3, 4) sur l'axe principal (10) et fait partie du dispositif de fixation pour la fibre optique.

6. Pièce à main (1) selon la revendication 5 pour autant qu'elle se réfère à la revendication 4, le joint (5) étant agencé sur un côté, opposé au tube de guidage (9), de la tubulure d'alimentation (12) en liquide de refroidissement.

7. Pièce à main (1) selon l'une quelconque des revendications précédentes, le tube de guidage (9) étant enfiché dans une tubulure de réception (11) du tube de guidage dépassant du corps de préhension (2).

8. Pièce à main (1) selon l'une quelconque des revendications précédentes, un tuyau d'aspiration (14) élastique, dont le diamètre interne est supérieur au diamètre externe du tube de guidage (9), étant agencé sur le tube de guidage (9).

9. Pièce à main (1) selon la revendication 8, une tubulure d'aspiration (15) en communication avec le tuyau d'aspiration (14) étant fixée sur le corps de préhension (2) ou sur un répartiteur d'aspiration séparé glissé sur le tube de guidage (9).

10. Pièce à main (1) selon l'une quelconque des revendications précédentes, le tube de guidage (9) étant réalisé en Nitinol.

11. Pièce à main (1) selon l'une quelconque des revendications précédentes, tous les éléments élastiques étant réalisés en caoutchouc de silicone.

12. Pièce à main (1) selon l'une quelconque des revendications précédentes, le corps de préhension (2) ou les parties de préhension (3, 4) étant réalisé(es) en PEEK.

13. Pièce à main (1) selon l'une quelconque des revendications 1 à 11, le corps de préhension (2) ou les parties de préhension (3, 4) étant réalisé(es) en aluminium revêtu.

14. Pièce à main (1) selon la revendication 13, l'aluminium étant sablé aux billes de verre et/ou revêtu de DLC.

15. Pièce à main (1) selon l'une quelconque des revendications précédentes,
- la tubulure d'alimentation (12) en liquide de refroidissement étant réalisée en PEEK et/ou
- la tubulure de réception (11) du tube de guidage étant réalisée en PEEK ou en acier inoxydable et/ou
- la tubulure d'aspiration (15) étant réalisée en PEEK ou en caoutchouc de silicone.
